# EUROPÄISCHE PATENTANMELDUNG

(11) **EP 2 366 374 A2**
(43) Veröffentlichungstag der Anmeldung: **21.09.2011**
(21) Anmeldenummer: 11153734.6
(22) Anmeldetag: 08.02.2011
(51) Int. Cl.: A61K 8/14, A61K 8/73, A61Q 17/04

(54) **UV-Schutzmittel für die Haut oder die Haare mit einer in einem vesikulären Trägersystem verkapselten UV-Filtersubstanz**

(30) Priorität: 12.02.2010 DE 102010001905
(71) Anmelder: ROVI Cosmetics International GmbH, 36381 Schlüchtern (DE)
(72) Erfinder: Teichmüller, Dirk, 63589, Linsengericht (DE); Beyer, Monika, 61130, Nidderau-Ostheim (DE); Sacher, Michael, 36381, Schlüchtern (DE)
(74) Vertreter: WSL Patentanwälte

(57) **Zusammenfassung**

Um ein UV-Schutzmittel für die topische Applikation auf die Haut oder die Haare mit sehr hoher UV-Filtersubstanzkonzentration und mit einer guten Adhäsion an Haut und Haar bereitzustellen, wird erfindungsgemäß ein UV-Schutzmittel mit wenigstens einer UV-Filtersubstanz, die in einem vesikulären Trägersystem verkapselt ist, vorgeschlagen, wobei das UV-Schutzmittel dadurch gekennzeichnet ist, dass die wenigstens eine in dem vesikulären Trägersystem verkapselte UV-Filtersubstanz in Öl löslich ist und das vesikuläre Trägersystem aus Vesikeln besteht, die aus hydrophobierten Polysacchariden aufgebaut sind und eine Teilchengröße von 10 bis 1000 nm sowie eine positive Oberflächenladung mit einem Zetapotential in dem Bereich von 1 bis 150 mV aufweisen. Desweiteren wird die Verwendung eines solchen UV-Schutzmittels in entsprechenden kosmetischen und/oder pharmazeutischen Formulierungen vorgeschlagen.

## Beschreibung

Die vorliegende Erfindung betrifft ein UV-Schutzmittel für die topische Applikation auf die Haut oder die Haare mit wenigstens einer UV-Filtersubstanz, die in einem vesikulären Trägersystem verkapselt ist, sowie die Verwendung eines solchen UV-Schutzmittels in entsprechenden kosmetischen und/oder pharmazeutischen Formulierungen.

Das für das menschliche Auge sichtbare Sonnenlicht erstreckt sich über ein Strahlungsspektrum von 400 bis 800 nm. Unterhalb von 400 nm liegt das Spektrum der UV-Strahlung (UV=Ultraviolett). Obwohl die Ultraviolettstrahlung die niederenergetischste ionisierende Strahlungsform darstellt, kann sie für dieser Strahlung exponierte Körperoberflächen schädlich sein.

Auf der Haut bewirken UV-A-Strahlen (320 bis 400 nm) u.a. eine Schädigung der Kollagene, wodurch die Haut an Spannkraft verliert und daher frühzeitig altern kann. Außerdem führt erhöhte Strahlenbelastung im UV-A-Bereich zu einem höheren Melanomrisiko. UV-B-Strahlen (280 bis 320 nm) führen zu entzündungsbedingter Hautrötung und verursachen den allseits bekannten Sonnenbrand.

Für die Haare geht mit der UV-Bestrahlung ein photochemisch induzierter Verlust von Proteinen und der Abbau des Haarfarbstoffs Melanin einher. UV-B-Strahlen sorgen dabei vor allem für morphologische Schäden am Haar, wie z.B. den Abbau von Haarproteinen, während UV-A-Strahlen besonders biochemische Veränderungen und Farbveränderungen verursachen können. Aminosäuren, aus denen die Haarproteine zusammengesetzt sind, sind lichtempfindlich. Ihre photochemische Schädigung produziert freie Radikale, die ihrerseits die Proteinstruktur des Keratins und damit die Haare weiter schädigen.

Es besteht daher ein Bedarf nach UV-Schutzmitteln, um sich gegen die vorgenannten schädlichen Wirkungen des UV-Anteils im Sonnenlicht zu schützen. Solche UV-Schutzmittel werden üblicherweise auf die Haut oder auf die Haare aufgetragen, um die negativen Wirkungen der Sonnenstrahlung (wie Sonnenbrand, Hautalterung, Schädigung des Haares) zu verhindern. Hierfür enthalten die genannten UV-Schutzmittel geeignete UV-Filtersubstanzen.

Bei den UV-Filtersubstanzen wird zwischen chemischen und physikalischen UV-Filtersubstanzen unterschieden. Chemische Filtersubstanzen absorbieren energiereiche Strahlung und geben sie als energieärmere, langwelligere Strahlung oder Wärme wieder ab. Physikalische Filtersubstanzen dagegen streuen und reflektieren das Licht hauptsächlich. Da die einzelnen Substanzen in der Regel keinen Schutz über das gesamte UV-Spektrum hinweg bieten, werden meist mehrere Stoffe kombiniert.

Um einen optimalen UV-Schutz zu erlangen, muss eine möglichst hohe UV-Filtersubstanzkonzentration in einem UV-Schutzmittel vorliegen. Dies ist bei vielen herkömmlichen UV-Schutzmitteln nur bedingt in dem gewünschten Maße zufriedenstellend gewährleistet. Insbesondere werden die für einen optimalen UV-Schutz gewünschten Konzentrationen häufig dann nicht erreicht, wenn eine Kombination von zwei oder mehr verschiedenen UV-Filtersubstanzen mit synergistischem Wirkspektrum in jeweils hoher Konzentration erforderlich ist, um ein UV-Schutzmittel mit einem optimalen Breitbandschutz über verschiedene Bereiche des relevanten UV-Spektrums zu erreichen.

Der vorliegenden Erfindung liegt daher die Aufgabe zugrunde, ein UV-Schutzmittel bereitzustellen, in dem UV-Filtersubstanz in sehr hohen Konzentrationen enthalten sein kann. Insbesondere soll es möglich sein zwei oder mehr verschiedene UV-Filtersubstanzen mit synergistischem Wirkspektrum in jeweils sehr hoher Konzentration in das UV-Schutzmittel stabil einzubringen, um ein UV-Schutzmittel mit einem optimalen Breitbandschutz über verschiedene Bereiche des relevanten UV-Spektrums zu erreichen. Desweiteren soll die Möglichkeit bestehen, Stoffe, die in der Lage sind, UV-Filtersubstanzen vor Zersetzung durch UV-Strahlung zu schützen und sie somit zu stabilisieren, sogenannte Photostabilisatoren, zusammen mit den UV-Filtersubstanzen in das UV-Schutzmittel stabil zu integrieren.

Darüber hinaus ist es gewünscht, dass das UV-Schutzmittel mit den vorgenannten Fähigkeiten auch eine gute Adhäsion an Haut und Haar bietet, um damit eine möglichst lange Verweildauer der UV-Filtersubstanz bzw. der UV-Filtersubstanzen auf der Haut oder den Haaren zu gewährleisten. Insbesondere soll die Adhäsion so effektiv sein, dass die UV-Filtersubstanz bzw. die UV-Filtersubstanzen auch mehrere Waschgänge auf der Haut bzw. den Haaren überdauern kann.

Diese Aufgabe wird erfindungsgemäß gelöst durch ein UV-Schutzmittel für die topische Applikation auf die Haut oder die Haare mit wenigstens einer UV-Filtersubstanz, die in einem vesikulären Trägersystem verkapselt ist, wobei das UV-Schutzmittel dadurch gekennzeichnet ist, dass die wenigstens eine in dem vesikulären Trägersystem verkapselte UV-Filtersubstanz in Öl löslich ist und das vesikuläre Trägersystem aus Vesikeln besteht, die aus hydrophobierten Polysacchariden aufgebaut sind und eine Teilchengröße von 10 bis 1000 nm sowie eine positive Oberflächenladung mit einem Zetapotential in dem Bereich von 1 bis 150 mV aufweisen.

Es konnte gezeigt werden, dass die erfindungsgemäßen UV-Schutzmittel den Vorteil bieten, dass große Mengen öllöslicher UV-Filtersubstanz stabil in ein nano-skaliges, neuartiges Trägersystem eingebracht werden können und so die Wirkung des UV-Schutzmittels optimieren.

Durch die Verkapselung in einem positiv geladenen Trägersystem kann zum einen die Adhäsion an Haut und Haar und damit die Verweildauer der UV-Filtersubstanz signifikant gesteigert werden. Die erfindungsgemäßte Zubereitung zeigt in dieser Hinsicht nachweislich eine gute Filmbildung auf Haut und Haar, sowie eine mehrere Waschgänge überdauernde effektive Adhäsion. Sowohl die nach mehreren Waschgängen auf Haut und Haar verbleibende Menge an UV-Filtersubstanz als auch deren Schutzwirkung konnten durch die erfindungsgemäßte Zubereitung gegenüber freien UV-Filtersubstanzen in erheblichem Maße gesteigert werden.

Zum anderen wird durch die Verkapselung der UV-Filtersubstanz eine Einarbeitung in hoher und wirksamer Konzentration in hydrophile kosmetische und/oder pharmazeutische Formulierungen ermöglicht. Auch die Kombination mehrerer öl-löslicher UV-Filtersubstanzen mit synergistischem Wirkspektrum in einem Trägersystem ist durch ein UV-Schutzmittel mit den erfindungsgemäßen Eigenschaften möglich. Auf diese Weise kann ein äußerst effektiver Breitbandschutz über den UVA- und UVB-Bereich erreicht werden.

Der Begriff "Verkapselung" umfasst im Zusammenhang mit der vorliegenden Erfindung die prinzipielle Einbettung von UV-Filtersubstanzen zwischen den Polysaccharidmolekülen und den von den Polysaccharidmolekülen ausgebildeten Nanostrukturen sowie den Einschluss der UV-Filtersubstanzen im Vesikelinneren.

Die erfindungsgemäßen Lipidvesikel haben ein Zetapotential im Bereich von 1 bis 150 mV. Der Begriff "Zetapotential" beschreibt das elektrische Potential einer Abscherschicht eines bewegten Partikels in einer Suspension. Die Messung des Zetapotentials kann dadurch erfolgen, dass man Partikel durch ein angelegtes elektrisches Feld bewegt. Aus der resultierenden Geschwindigkeit der Partikel lässt sich dann das Zetapotential berechnen. Bevorzugte Lipidvesikel weisen ein Zetapotential von 30 bis 100 mV auf. Bei besonders bevorzugten Lipidvesikeln beträgt das Zetapotential 40 bis 60 mV.

Die verbesserte Adhäsion des UV-Schutzmittels basiert u.a. auf der positiven Ladung der Vesikeloberfläche. Die positive Ladung der Vesikeloberfläche führt zu einer verbesserten Anhaftung der Vesikel an der Oberfläche von Hautzellen. Darüber hinaus führt die positive Ladung der Vesikel auch zu einer starken Anhaftung an der Haaroberfläche. Es hat sich außerdem gezeigt, dass diese verbesserte Adhäsion auch mit der erfindungsgemäß nano-skaligen Teilchengröße der Trägervesikel zusammenhängt, wobei all diese Feststellungen in Bezug auf die vorliegende Erfindung jedoch nicht bindend sein sollen und den Umfang der Erfindung daher auch nicht beschränken sollen.

Die Teilchengröße der erfindungsgemäßen Lipidvesikel ist in dem Bereich von 10 bis 1000 nm. Bei bestimmten Ausführungsformen ist die Teilchengröße 100 bis 400 nm. Bei anderen Ausführungsformen ist sie 100 bis 350 nm. Bei bevorzugten Ausführungsformen ist die Teilchengröße 100 bis 250 nm.

Der Begriff "Teilchengröße" bedeutet im Zusammenhang mit der vorliegenden Erfindung die mittlere Teilchengröße. Die mittlere Teilchengröße ist mittels Photonenkorrelationsspektroskopie bestimmbar. Die Photonenkorrelationsspektroskopie - auch als dynamische Lichtstreuung bezeichnet - ist ein optisches Messverfahren zur Bestimmung der Größenverteilung von Vesikeln und Partikeln in Flüssigkeiten. Die Methode nutzt die Streuung von Laserlicht durch die Vesikel aus.

Durch die erfindungsgemäße Kombination von positiver Ladung der Vesikeloberfläche und nanoskaliger Teilchengröße der Trägervesikel wird sowohl bei Haut- als auch bei Haaranwendungen erreicht, dass in den Vesikeln enthaltene UV-Filtersubstanz/en dauerhafter auf den zu schützenden Oberflächen verbleiben.

Vorzugsweise wird die positive Ladung der Vesikeloberfläche dadurch bewirkt wird, dass die Vesikel zusätzlich zu den Polysacchariden, aus denen die Vesikel aufgebaut sind, positiv geladene Moleküle als Ladungsgeber aufweisen, wobei diese Ladungsgeber ausgewählt sind unter primären, sekundären, tertiären und quarternären Alkyl-Ammoniumsalzen oder Kombinationen davon. Besonders bevorzugt sind hierbei C₁₂-C₂₂- Alkyl-Ammoniumsalze.

Ganz besonders bevorzugt sind als Ladungsgeber bei der vorliegenden Erfindung quarternäre C₁₂-C₂₂-Alkyl-Trimethylammoniumsalze (bzw. Fettsäure-Trimoniumsalze) der Formel 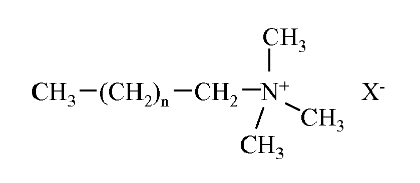 wobei n eine ganze Zahl von 10 bis 22 und X- ein anorganisches oder organisches Anion ist. Vorzugsweise ist n in der oben angegebenen Alkyl-Trimethylammoniumsalzformel gleich 22.

Vorzugsweise ist X- in der oben angegebenen Formel ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X- Bromid, Chlorid, Fluorid, Iodid, Saccharinat, Tosylat oder Methosulfat.

Vorzugsweise sind die Ladungsgeber bei der vorliegenden Erfindung ausgewählt unter einem oder mehreren von Ceteartrimoniumchlorid, Cetrimoniumbromid, Cetrimoniumchlorid, Cetrimoniumsaccharinat, Cetrimoniumtosylat, Cocotrimoniumchlorid, Cocotrimoniummethosulfat, Lautrimoniumbromid, Lautrimoniumchlorid, Myrtrimoniumbromid, Octyldecyltrimoniumchlorid, Steartrimoniumbromid, Steartrimoniumchlorid, Steartrimoniummethosulfat und Steartrimoniumsaccharinat. Bei einer besonders bevorzugten Ausführungsform der vorliegenden Erfindung wird Behentrimoniumchlorid als Ladungsgeber verwendet.

Vorzugweise werden bei der vorliegenden Erfindung einer oder mehrere der vorgenannten positiv geladenen Ladungsgeber so eingesetzt, dass der Ladungsgeberanteil in den Vesikeln bezogen auf das Gesamtgewicht der Vesikel insgesamt in dem Bereich von 0,01 bis 10 Gew.-% liegt. Bei bestimmten Ausführungsformen liegt der Ladungsgeberanteil in dem Bereich von 0,01 bis 2,0 Gew.-%.

Die in Gewichtsprozenten angegebenen Anteile beziehen sich prinzipiell auf die vollständige Formulierung des UV-Schutzmittels gemäß Rezepturvorgaben, inklusive des zur Vesikelbildung erforderlichen Wasseranteils, wenn nicht etwas anderes angegeben ist.

Der angegebene Anteil umfasst außerdem alle eindeutig unter die Definition der jeweiligen Substanzgruppe fallenden Substanzen. Demnach umfasst der angegebene Ladungsgeberanteil bei den Ausführungsformen mit einem Ladungsgeber den Anteil dieses einen Ladungsgebers und bei Ausführungsformen mit mehreren Ladungsgebern die Summe aller Ladungsgeber. Die gilt im Übrigen in gleicher Weise für im Zusammenhang mit dieser Erfindung angegebene Anteile an hydrophobiertem Polysaccharid, Filtersubstanz und Hilfsstoff oder anderen Substanz- bzw. Stoffanteilen.

Das Polysaccharidgrundgerüst der hydrophobierten Polysaccharide, die bei dem UV-Schutzmittel für die Bildung der Vesikel verwendet werden können, kann unter allen kosmetisch oder pharmazeutisch verträglichen Polysacchariden ausgewählt werden, die in der Lage sind, Vesikel zu bilden. Vorzugsweise handelt es sich um wasserlösliche Polysaccharide und/oder um deren Ether mit kurzkettigen Alkoholen (C₁ bis C₄), wobei die wasserlöslichen Polysaccharide linear, verzweigt, kammartig und/oder sternförmig sein können. Besonders bevorzugt sind lineare wasserlösliche Polysaccharide. Es kommen auch Copolymere oder Block-Copolymere unterschiedlicher Monosaccharid-Einheiten und/oder auf unterschiedliche Weise miteinander verknüpfter Monosaccharid-Einheiten in Betracht.

Die hydrophobierten Polysaccharide, aus denen die Vesikel aufgebaut sind, weisen vorzugsweise ein Polysaccharid-Grundgerüst aus Polyglucose oder Polyfructose auf. Bevorzugte hydrophobierte Polysaccharide mit einem Polysaccharid-Grundgerüst aus einer Polyglucose sind Cellulose, Methylcellulose, Hydroxyethylcellulose, Amylose, Amylopektin und Dextrin. Ein bevorzugtes hydrophobiertes Polysaccharid mit einem Polysaccharid-Grundgerüst aus Polyfructose ist Inulin.

Das hydrophobierte Polysaccharid umfasst bei der vorliegenden Erfindung vorzugsweise im Mittel von 5 bis 1000 Monosaccharid-Einheiten. Noch bevorzugter sind von 10 bis 500 Monosaccharid-Einheiten. Insbesondere bevorzugt sind von 20 bis 100 Monosaccharid-Einheiten.

Bei speziellen Ausführungsformen können auch Gemische der oben genannten Polysaccharide eingesetzt werden mit der Maßgabe, dass diese Gemische in der Lage sind, Vesikel zu bilden.

Der Anteil der Polysaccharide in den Vesikeln liegt bezogen auf das Gesamtgewicht der Vesikel in dem Bereich von 1 bis 85 Gew.-%. Vorzugsweise beträgt der Polysaccharidanteil bezogen auf das Gesamtgewicht der Vesikel 5 bis 25 Gew.-%. Besonders bevorzugt beträgt der Polysaccharidanteil 8 bis 15 Gew.-%.

Bei bevorzugten Ausführungsformen sind die hydrophobierten Polysaccharide, aus denen die Vesikel aufgebaut sind, dadurch hydrophobiert, dass sie ein Polysaccharid-Grundgerüst mit C₃₋₂₂-Alkylgruppen, die über Alkyl-Etherbindungen oder über Alkyl-Urethanbindungen an die Hydroxygruppen des Polysaccharids gebunden sind, oder die über einen Linker (z.B. Polyether-Linker, Polyethylenglykol-Linker) an das Polysaccharid-Grundgerüst gebunden sind, aufweisen.

Das Molekulargewicht des hydrophob modifizierten Polysaccharids liegt bei bevorzugten Ausführungsformen in dem Bereich von 5000 bis 500.000 g/mol. Bevorzugt ist hierbei der Bereich von 5000 bis 100.000 g/mol.

Bei bevorzugten hydrophob modifizierten Polysacchariden beträgt der Quotient aus Anzahl an hydrophob modifizierenden Gruppen und modifizierbaren Gruppen (Modifizierungsgrad) von 0,01 bis 0,9. Bei besonders bevorzugten Ausführungsformen beträgt der Modifizierungsgrad von 0,03 bis 0,15.

Die hydrophoben Gruppen und auch das Polysaccharid-Rückgrat können bei bestimmten Ausführungsformen durch beispielsweise Halogen-, Hydroxy-, Alkoxy-, Amino-, Alkylamino-, Aryl-, Arylalkyl-, Carboxy-, Carboxyester- und cycloaliphatische Reste ein- oder mehrfach substituiert sein. Bevorzugt handelt es sich bei den hydrophob modifizierten Polysacchariden jedoch um nichtionische Verbindungen.

Erfindungsgemäß besonders bevorzugt eingesetzte hydrophob modifizierte Polysaccharide sind Inulinlaurylcarbamate, Cetylhydroxyethylcellulose, Hydroxy-C3-6-alkyl-modifizierte Cellulose, insbesondere Hydroxypropylcellulose und Hydroxypropylmethylcellulose.

Als UV-Filtersubstanz kommen bei der vorliegenden Erfindung uneingeschränkt alle chemisch oder physikalisch UV-filternd wirkenden Substanzen in Betracht, vorausgesetzt, sie sind in Öl löslich. Die chemischen Filtersubstanzen absorbieren energiereiche Strahlung und geben sie als energieärmere, langwelligere Strahlung oder Wärme wieder ab. Die physikalischen Filtersubstanzen streuen und reflektieren das Licht hauptsächlich.

Bevorzugte Beispiele für UV-Filtersubstanzen, die bei der vorliegenden Erfindung zur Anwendung kommen können, sind ohne Beschränkung hierauf 3-Benzylidencampher, 4-Methylbenzylidencampher, Benzophenon-1, Benzophenon-2, Benzophenon-3, Benzophenon-4, Benzophenon-5, Benzophenon-6, Benzophenon-9, Benzylidencamphersulfonsäure, Bis-Ethylhexyloxyphenolmethoxyphenyltriazin, Butylmethoxydibenzoylmethan, Campherbenzalkoniummethosulfat, Diethylaminohydroxybenzoylhexylbenzoat, Diethylhexylbutamidotriazon, Dinatriumphenyldibenzimidazoltetrasulfonat, Drometrizoltrisiloxan, Ethylhexyldimethylpaba, Ethylhexylmethoxycinnamat, Ethylhexylsalicylat, Ethylhexyltriazon, Homosalat, Isoamyl-p-methoxycinnamat, Methylen-bis-benzotriazolyltetramethylbutylphenol, Octocrylen, PEG-25-Paba, Phenylbenzimidazolsulfonsäure, Polyacrylamidomethylbenzylidencampher, Polysilicon-15, Kalium-phenylbenzimidazolsulfonat, Natriummangoseedat, Natriumphenylbenzimidazolsulfonat, Tea-Phenylbenzimidazolsulfonat, Terephthalylidendicamphersulfonsäure, Ferulasäure, Cinoxat, Diisopropylmethylcinnamat, 4-(2-Beta-Glucopyranosiloxy)propoxy-2-hydroxybenzophenon, Glycerylethylhexanoatdimethoxycinnamat, Isopentyltrimethoxycinnamattrisiloxan.

Vorzugsweise beträgt der Anteil an UV-Filtersubstanz bei der vorliegenden Erfindung 1 bis 65 Gew.-% bezogen auf das Gesamtgewicht der vollständigen Formulierung des UV-Schutzmittels gemäß Rezepturvorgaben, inklusive des zur Vesikelbildung erforderlichen Wasseranteils.

Da die einzelnen Filtersubstanzen in der Regel keinen Schutz über das gesamte UV-Spektrum hinweg bieten, werden bei bevorzugten Ausführungsformen der Erfindung mehrere Filtersubstanzen kombiniert, um einen möglichst breiten UV-Schutz zu erreichen.

Erfindungsgemäß wird das UV-Schutzmittel in kosmetischen und/oder pharmazeutischen Formulierungen verwendet, wobei pharmazeutische Formulierungen solche sind, die unter das Arzneimittelrecht fallen.

Die Formulierungen können alle Hilfs- und Zusatzstoffe, die üblicherweise bei kosmetischen oder pharmazeutischen Präparaten Anwendung finden, enthalten. Insbesondere fallen unter den Begriff "Hilfsstoff" im Zusammenhang mit der vorliegenden Erfindung solche Zusatzstoffe, die auf die physikalischen Eigenschaften der Vesikel und deren Stabilität einwirken und/oder der Konservierung der UV-Schutzmittel dienen. Beispiele für solche Hilfsstoffe sind Öle, Alkohole, Polyole, Antioxidantien, Gelbildner, Puffer, Konservierungsmittel, Bakterizide und Keimhemmer, Konsistenzgeber, Verdicker und Komplexbildner.

Vorzugsweise ist das UV-Schutzmittel gemäß der vorliegenden Erfindung dadurch gekennzeichnet, dass der Hilfsstoffanteil bezogen auf das Gesamtgewicht der vollständigen Formulierung des UV-Schutzmittels gemäß Rezepturvorgaben, inklusive des zur Vesikelbildung erforderlichen Wasseranteils in dem Bereich von 0,01 bis 10 Gew.-% liegt.

Bevorzugt wird das erfindungsgemäße UV-Schutzmittel für kosmetische und/oder pharmazeutische Formulierungen verwendet, die zur topischen Applikation geeignet sind. Das erfindungsgemäße UV-Schutzmittel kann hierfür in allen für die topische Applikation geeigneten Formulierungen vorliegen, beispielsweise in Form eines Gels, einer Creme, einer Salbe, eines Sprays oder einer Lotion. Dazu kann das erfindungsgemäße UV-Schutzmittel in eine Trägermatrix eingearbeitet werden. Bei der Trägermatrix kann es sich um Gelformulierungen, Cremeformulierungen, Lotionen, Maskenanwendungen etc. handeln.

Für Anwendungen im Hautbereich wird das erfindungsgemäße UV-Schutzmittel vorzugsweise in einer Lotion, einer Creme, einer Salbe, einem Gel, einem wässrigen Fluid, einem Gesichtswasser, einem Sonnenprodukt oder einer Maske angewendet.

Für Anwendungen im Haarbereich wird das erfindungsgemäße UV-Schutzmittel vorzugsweise in einem Shampoo (vorzugsweise mit milden Tensiden), einer Spülung, einem Haargel, einem Conditioner, einem Hair Tonic, einem Haar-Styling-Produkt oder einem Haar- Pflege-Produkt angewendet.

Selbstverständlich handelt es sich bei allen Komponenten des erfindungsgemäßen UV-Schutzmittels und von dessen Formulierungen um pharmazeutisch, kosmetisch oder dermatologisch verträgliche Stoffe. Ein Stoff ist im Sinne dieser Erfindung pharmazeutisch, kosmetisch oder dermatologisch verträglich, wenn er nicht toxisch ist und bei der Mehrzahl der potentiellen Anwender topisch anwendbar ist, ohne dass es bei dem Anwender spontan oder nach einer Weile zu einer ungewünschten physiologischen Reaktion, wie z.B. einer Rötung oder der Ausbildung eines Juckreizes kommt.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische, pharmakologische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

## Patentansprüche

1. UV-Schutzmittel für die topische Applikation auf die Haut oder die Haare mit wenigstens einer UV-Filtersubstanz, die in einem vesikulären Trägersystem verkapselt ist, **dadurch gekennzeichnet, dass** die wenigstens eine UV-Filtersubstanz in Öl löslich ist und das vesikuläre Trägersystem aus Vesikeln besteht, die aus hydrophobierten Polysacchariden aufgebaut sind und eine Teilchengröße von 10 bis 1000 nm sowie eine positive Oberflächenladung mit einem Zetapotential in dem Bereich von 1 bis 150 mV aufweisen.

2. UV-Schutzmittel gemäß Anspruch 1, **dadurch gekennzeichnet, dass** der UV-Filtersubstanzanteil in den Vesikeln bezogen auf das Gesamtgewicht des UV-Schutzmittels in dem Bereich von 1 bis 65 Gew.-% liegt.

3. UV-Schutzmittel gemäß Anspruch 2, **dadurch gekennzeichnet, dass** der Polysaccharidanteil in den Vesikeln bezogen auf das Gesamtgewicht der Vesikel in dem Bereich von 1 bis 85 Gew.-% liegt.

4. UV-Schutzmittel gemäß einem der vorangehenden Ansprüche, **dadurch gekennzeichnet, dass** die positive Oberflächenladung dadurch bewirkt wird, dass die Vesikel zusätzlich zu den Polysacchariden, aus denen die Vesikel aufgebaut sind, positiv geladene Moleküle als Ladungsgeber aufweisen, wobei diese Ladungsgeber ausgewählt sind unter primären, sekundären, tertiären und quarternären Alkyl-Ammoniumsalzen oder Kombinationen davon.

5. UV-Schutzmittel gemäß Anspruch 4, **dadurch gekennzeichnet, dass** der Ladungsgeberanteil in den Vesikeln bezogen auf das Gesamtgewicht des UV-Schutzmittels in dem Bereich von 0,01 bis 10 Gew.-% liegt.

6. UV-Schutzmittel gemäß einem der vorangehenden Ansprüche, **dadurch gekennzeichnet, dass** die hydrophobierten Polysaccharide, aus denen die Vesikel aufgebaut sind, ein Polysaccharid-Grundgerüst aus Polyglucose oder Polyfructose aufweisen.

7. UV-Schutzmittel gemäß einem der vorangehenden Ansprüche, **dadurch gekennzeichnet, dass** die hydrophobierten Polysaccharide, aus denen die Vesikel aufgebaut sind, ein Polysaccharid-Grundgerüst aus einer Polyglucose, die unter Cellulose, Methylcellulose, Hydroxyethylcellulose, Amylose, Amylopektin oder Dextrin ausgewählt ist, oder ein Polysaccharid-Grundgerüst aus der Polyfructose Inulin aufweisen.

8. UV-Schutzmittel gemäß einem der vorangehenden Ansprüche, **dadurch gekennzeichnet, dass** die hydrophobierten Polysaccharide, aus denen die Vesikel aufgebaut sind, ein Polysaccharid-Grundgerüst aufweisen, das durch C₃-₂₂-Alkylgruppen, die über Alkyl-Etherbindungen oder über Alkyl-Urethanbindungen an die Hydroxygruppen des Polysaccharids gebunden sind, oder die über einen Linker an das Polysaccharid-Grundgerüst gebunden sind, hydrophob modifiziert sind.

9. UV-Schutzmittel gemäß einem der vorangehenden Ansprüche, **dadurch gekennzeichnet, dass** die wenigstens eine UV-Filtersubstanz eine chemische oder physikalische UV-Filterwirkung aufweist.

10. UV-Schutzmittel gemäß einem der vorangehenden Ansprüche, **dadurch gekennzeichnet, dass** die wenigstens eine UV-Filtersubstanz ausgewählt ist unter 3-Benzylidencampher, 4-Methylbenzylidencampher, Benzophenon-1, Benzophenon-2, Benzophenon-3, Benzophenon-4, Benzophenon-5, Benzophenon-6, Benzophenon-9, Benzylidencamphersulfonsäure, Bis-Ethylhexyloxyphenolmethoxyphenyltriazin, Butylmethoxydibenzoylmethan, Campherbenzalkoniummethosulfat, Diethylaminohydroxybenzoylhexylbenzoat, Diethylhexylbutamidotriazon, Dinatriumphenyldibenzimidazoltetrasulfonat, Drometrizoltrisiloxan, Ethylhexyldimethylpaba, Ethylhexylmethoxycinnamat, Ethylhexylsalicylat, Ethylhexyltriazon, Homosalat, Isoamyl-p-methoxycinnamat, Methylen-bis-benzotriazolyltetramethylbutylphenol, Octocrylen, PEG-25-Paba, Phenylbenzimidazolsulfonsäure, Polyacrylamidomethylbenzylidencampher, Polysilicon-15, Kaliumphenylbenzimidazolsulfonat, Natriummangoseedat, Natriumphenylbenzimidazolsulfonat, Tea-Phenylbenzimidazolsulfonat, Terephthalylidendicamphersulfonsäure, Ferulasäure, Cinoxat, Diisopropylmethylcinnamat, 4-(2-Beta-Glucopyranosiloxy)propoxy-2-hydroxybenzophenon, Glycerylethylhexanoatdimethoxycinnamat, Isopentyltrimethoxycinnamattrisiloxan.

11. UV-Schutzmittel gemäß einem der Ansprüche 1 bis 10, **dadurch gekennzeichnet, dass** es zusätzlich einen oder mehrere Hilfsstoffe umfasst.

12. UV-Schutzmittel gemäß einem der Ansprüche 1 bis 11, **dadurch gekennzeichnet, dass** der Hilfsstoffanteil in den Vesikeln bezogen auf das Gesamtgewicht des UV-Schutzmittels in dem Bereich von 0,01 bis 10 Gew.-% liegt.

13. Verwendung eines UV-Schutzmittels gemäß einem der Ansprüche 1 bis 12 zur Herstellung einer kosmetischen und/oder pharmazeutischen Formulierung.

14. Kosmetische und/oder pharmazeutische Formulierung, die ein UV-Schutzmittel gemäß einem der Ansprüche 1 bis 12 enthält.
